# EUROPEAN PATENT SPECIFICATION

(11) **EP 0 669 400 B1**
(45) Date of publication and mention of the grant of the patent: **30.08.2000**
(21) Application number: 95101723.5
(22) Date of filing: 09.02.1995
(51) Int. Cl.: C12Q 1/68, C07K 14/47

(54) **Method of diagnosing cancer or precancerous state by analysis of IRF-1-specific RNA**
Verfahren zur Diagnose von Krebs oder Präkanzerose durch Nachweis IRF-1-spezifischer RNS
Méthode de diagnostic du cancer ou prénéoplasie par la détection d'ARN IRF-1

(30) Priority: 24.02.1994 EP 94102839
(43) Date of publication of application: 30.08.1995
(73) Proprietor: BOEHRINGER INGELHEIM INTERNATIONAL GmbH, 55218 Ingelheim am Rhein (DE)
(72) Inventor: Taniguchi, Prof. Dr. Tadatsugu, A207, Ibaraki-Shi, Osaka 567 (JP); Harada, Dr. Hisashi, Suita-shi, Osaka 565 (JP)

(56) References cited:
- EP-A- 0 571 743
- WO-A-91/02817
- WO-A-92/15602
- WO-A-94/06818
- SCIENCE, vol. 259,no. 5097, 12 February 1993 LANCASTER, PA US, pages 971-974, H.HARADA ET AL. 'Anti-oncogenic and oncogenic potentials of interferon regulatory factors-1 and -2'
- SCIENCE, vol. 259,no. 5097, 12 February 1993 LANCASTER, PA US, pages 968-971, C.L.WILLMAN ET AL. 'Deletion of IRF-1, mapping to chromosome 5q31.1, in human leukemia and preleukemic myelodysplasia'
- DNA AND CELL BIOLOGY, vol. 11,no. 8, 1992 NEW YORK US, pages 605-611, Y.CHA ET AL. 'Human interferon regulatory factor 1: intron-exon organization'
- PROCEEDINGS OF THE NATIONAL ACADEMY OF SCIENCES OF USA., vol. 90,no. 13, 1 July 1993 WASHINGTON US, pages 5893-5895, P.LENGYEL 'Tumor-suppressor genes: News about the interferon connection'

SEQUENCE LISTING

(1) GENERAL INFORMATION:

(i) APPLICANT:
(A) NAME: Boehringer Ingelheim International GmbH
(B) STREET: Binger Str.
(C) CITY: Ingelheim
(E) COUNTRY: Germany
(F) POSTAL CODE (ZIP): 55218
(G) TELEPHONE: (0)6132-772770
(H) TELEFAX: (0)6132-774377

(ii) TITLE OF INVENTION: Method of diagnosing cancer, precancerous
state, and susceptibility to other forms of diseases by
analysis of IRF-1-specific RNA in biopsy samples

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 1:

TTCCCTCTTC CACTCGGAGT 20

(2) INFORMATION FOR SEQ ID NO: 2:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 2:

GATATCTGGC AGGGAGTTCA 20

(2) INFORMATION FOR SEQ ID NO: 3:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 3:

CTCTTGGTCT TTCACCTCCT C 21

## Description

The present invention relates to a method of diagnosing cancer, precancerous state, and susceptibility to other forms of diseases, by use of a molecular marker, interferon-regulatory factor-1 (IRF-1).

### Technical background

Malignant cell transformation is a multistep process resulting from the progressive acquisition of structural alterations at multiple genetic loci which are involved in the regulation of cell growth. It has been well documented that gain-of-function mutations, found in dominantly-acting proto-oncogenes, are often accompanied by loss-of-function mutations in tumor suppressor genes in human malignant cells. Several tumor suppressor genes have been identified whose mutation or deletion appears to be critical for the development of human cancers, among them, *p53*, *RB* and *WT1*, whose gene products are found in nucleus and which function as regulators of gene transcription (reviewed in Kaelin, Jr., et al., 1991; Lewin, 1991; Marshall, 1991; Weinberg, 1991; Haber and Housman, 1992; Vogelstein and Kinzler, 1992; Levine, 1993),

Two structurally related transcription factors, IRF-1 and IRF-2 were originally identified as regulators of the interferon (IFN) system (Miyamoto et al., 1988; Harada et al., 1989; Tanaka and Taniguchi, 1992). IRF-1 has also been identified by others in different contexts (Pine et al., 1990; Yu-Lee et al., 1990; Abdollahi et al., 1991; Stark and Kerr, 1992). IRF-1 functions as a transcriptional activator whereas IRF-2 represses the effect of IRF-1 by competing for binding to the same DNA sequence elements (IRF-Es) (Harada et al., 1989; Tanaka et al., 1993). IRF-Es can be found in both the *IFN*-α and *IFN*-β promoters, as well as in IFN-stimulated response elements (ISREs) found within the promoters of IFN-inducible genes (Friedman and Stark, 1985; Shirayoshi et al., 1987; Levy et al., 1988). It has been shown that IRF-1 functions as an activator for the type I IFN genes and some IFN-inducible genes (Fujita et al., 1989; Harada et al., 1990; Au et al., 1992; Pine et al., 1992; Reis et al., 1992; Matsuyama et al., 1993; Ruffner et al., 1993; Kamijo et al., 1994).

Evidence has also been provided demonstrating a role for IRF-1 as a tumor suppressor; (i) IRF-1 manifests antiproliferative activities (Yamada et al., 1990; Kirchhoff et al., 1993; T. Tamura, M. S. L., and T. Kawakami, unpublished results), (ii) overexpression of the repressor IRF-2 in NIH 3T3 cells causes cell transformation and this cell transformation is suppressed by concomitant overexpression of the activator IRF-1 (Harada et al., 1993), and (iii) primary embryonic fibroblasts (EFs) with a null mutation in the *IRF-1* gene (IRF-1^{-/-} mice) are susceptible to transformation by an activated form of c-Ha-*ras*, a property also seen in the EFs from p53^{-/-} mice, but not in wild type EFs (Tanaka et al. 1994). These observations collectively suggest that the loss of IRF-1 function may contribute to the development of human neoplasia.

The human *IRF-1* gene has been mapped to 5q31.1 (Itoh et al., 1991; Willman et al., 1993; Harada et al., 1994). Chromosome band 5q31 was previously determined to be the most commonly deleted segment, the so-called "critical region", in human leukemia and MDS with interstitial deletions of chromosome 5q (Le Beau et al., 1986; Nimer and Golde, 1987; Le Beau et al., 1989; Pederson and Jensen, 1991). Del(5q) is also a hallmark of a unique clinical myelodysplastic disorder with refractory anemia and abnormal megakaryocytes occurring predominantly in elderly females, known as the "5q- Syndrome" (Van den Berghe et al., 1985). Hence it is believed that this chromosomal region harbors a tumor suppressor gene(s). However, in view of the variable clinical features of myeloid diseases associated with del(5q), it is possible that inactivation of such tumor suppressor gene(s) is accompanied by variable additional genetic events such as the activation of oncogenes in different types of myeloid disorders (Carter et al., 1992).

Previously, it had been demonstrated that one or both *IRF-1* alleles were deleted in each of 13 representative cases of MDS and leukemia with del(5q) or translocation of 5q31. Furthermore, inactivating gene rearrangement of one *IRF-1* allele, accompanied by deletion of the residual allele, were found in a case of *de novo* acute leukemia (Willman et al., 1993). These observations support the idea that *IRF-1* may be the critical tumor suppressor gene deleted in the del(5q); thus loss of one or both *IRF-1* alleles may contribute to unrestrained cellular proliferation thereby promoting the development of human leukemia and MDS. On the other hand, both *IRF-1* alleles are still retained in some MDS/leukemia patients exhibiting 5q deletion (Boultwood et al., 1993; Le Beau et al., 1993).

The problem underlying the present invention was to find a new method of diagnosing cancer by use of a molecular marker and to provide means for such a method.

### Disclosure of the invention

The new method of diagnosing cancer, precancerous state, or susceptibility to other forms of diseases provided by the present invention relies on the analysis of *IRF-1*-specific RNA in blood and other biopsy samples.

The invention is based on the unexpected finding that a novel mechanism of inactivation of the tumor suppressor gene *IRF-1* seems to play a crucial role in tumors and precancerous states, especially in diseases of the hematopoietic system. This mechanism appears to be independent of the previously described deletions or mutations of the *IRF-1* gene as it takes place in cases where such alterations are not detectable. The inactivation occurs through an altered splicing pattern of the primary transcript, hence on the RNA level. More specifically, RNA molecules lacking exon 2 or exons 2 and 3 can be detected in samples from either healthy donors or patients suffering from leukemia or myelodysplastic syndrome. These altered transcripts do not lead to functional IRF-1 polypeptide. The susceptibility to this unusual exon skipping seems to be an intrinsic property of the human *IRF-1* gene. Excitingly, the ratio between full-length transcripts and transcripts lacking exon 2 or exons 2 and 3 is significantly changed in a great number of patients with precancerous states or malignancies of the hematopoietic system as compared to healthy donors. Thus, the altered splicing pattern of *IRF-1* transcripts can serve as a molecular marker for malignant diseases, especially leukemia, or precancerous states, especially myelodysplastic syndrome, or susceptibility to other forms of diseases.

Accordingly, an aspect of the present invention is a method of diagnosing cancer, precancerous states, or susceptibility to other forms of diseases wherein it is determined whether the level of full-length *IRF-1*-RNA in a sample is reduced as compared to healthy donors. Another aspect is a method wherein it is determined whether *IRF-1*-specific RNA molecules occuring in a sample lack exon 2 or exons 2 and 3 of the *IRF-1* gene. Preferably, the relative amounts of full-length *IRF-1*-RNA, *IRF-1Δ2*-RNA, and *IRF-1Δ23*-RNA are determined.

The new method of diagnosing cancer, precancerous states, or susceptibility to other forms of diseases provided by the present invention relies on the analysis of *IRF-1*-specific RNA in blood or other biopsy samples. The term "*IRF-1*-specific RNA molecules" means RNA molecules which are originally generated by transcription of all or part of the *IRF-1* gene. This term applies also to such RNA molecules which are later modified, e.g. by splicing. Preferably, said analysis is carried out by polymerase chain reaction (PCR) employing *IRF-1*-specific primers, especially in combination with a reverse transcription (RT) step preceding the amplification step. The term "*IRF-1*-specific oligonucleotide" shall mean any oligonucleotide having a nucleotide sequence complementary to the sense or anti-sense strand of a part of the nucleotide sequence of the *IRF-1*-gene or *IRF-1* cDNA, or being capable of specifically hybridising to the *IRF-1*-specific RNA under the conditions of the PCR. Preferred primers for the PCR have the sequence
5'TTCCCTCTTCCACTCGGAGT3' (SEQ ID NO:1) or
5'GATATCTGGCAGGGAGTTCA3' (SEQ ID NO:2).
A primer which can be used well for reverse transcription has the sequence
5 'CTCTGGTCTTTCACCTCCTC3' (SEQ ID NO: 3).

It should be noted that the specific primers disclosed above are mere examples and can be substituted by other appropriate oligonucleotides.

Besides RT/PCR, other methods of analyzing RNA may be used as well, for example RT in combination with ligase chain reaction (LCR).

The sample which is to be investigated may be any biopsy or tissue sample, for example a sample derived from peripheral blood or bone marrow.

Preferably, the cancer, precancerous state, or other form of disease to be diagnosed is a diesease of the hematopoietic system, especially myelodysplasia or leukemia.

In another aspect, the present invention comprises *IRF-1*-specific oligonucleotides, preferably those having the sequences with SEQ ID NOs. 1, 2 or 3, and their use in the above methods.

Furthermore, the content of biologically active IRF-1 polypeptide may be determined in a sample obtained from blood or other biopsy material by an appropriate biological or immunological assay for diagnostic purposes.

### Exon skipping in the human IRF-1 gene

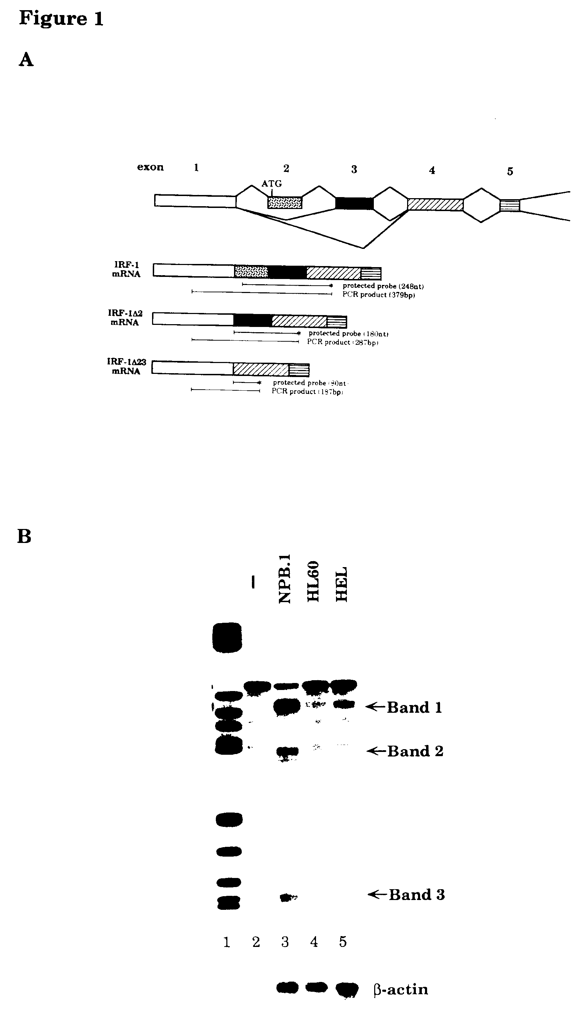

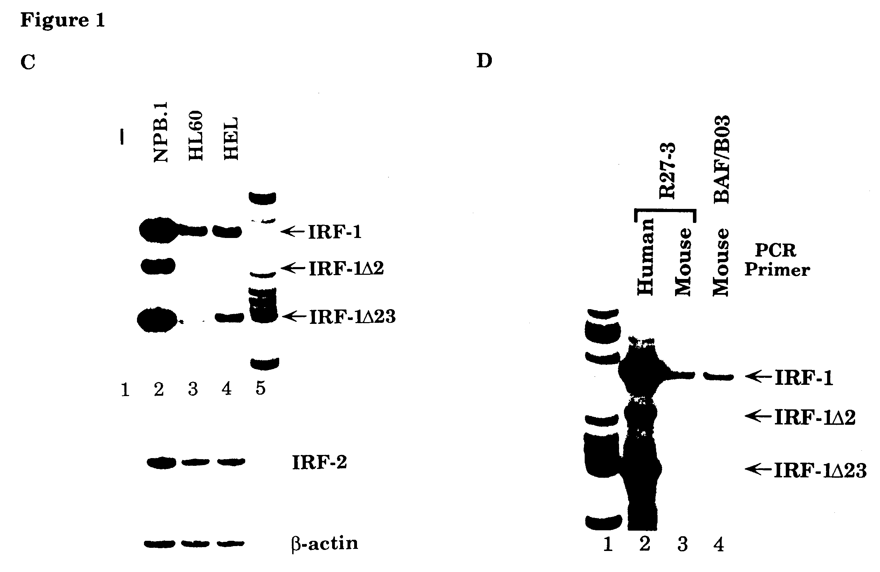

The human *IRF-1* gene consists of 10 exons, with the initiator ATG sequence located within the second exon (Figure 1A; Cha et al., 1993; Harada et al., 1994). During an analysis of *IRF-1* mRNA expression in various cell lines by S1 mapping, we noted the expression of unusual transcripts. As shown in Figure 1B, three protected bands were detected using total RNA from the peripheral blood mononuclear (PBM) cells of a healthy donor¹. The upper band (Band 1; 248 nucleotides) corresponds to the intact *IRF-1* mRNA. Judging by their size, the other two bands may correspond to RNAs lacking exon 2 (Band 2; 180 nucleotides), and exons 2 and 3 (Band 3; 80 nucleotides), respectively. However, these results were somewhat obscure in two hematopoietic cell lines, HL-60 and HEL, due to low mRNA expression levels. In the case of HL-60, this may be explained by the lack of one *IRF-1* allele, since the cell has lost one chromosome 5 (Gallagher et al., 1979). In order to achieve a more quantitative and qualitative analysis of these RNAs, we next performed a polymerase chain reaction (PCR) assay. In this assay, reverse transcription of mRNA was followed by PCR amplification (RT-PCR), in which the PCR products (amplicons) were labeled to high specific activity. As shown in Figure 1C, three bands were again detected with the RNA from PBM cells, diagnostic for the Bands 1, 2 and 3 which were generated by the S1 mapping analysis (Figure 1A). Nucleotide sequence analysis of the PCR-amplified cDNAs revealed that the upper, middle and lower bands in fact correspond to intact *IRF-1* mRNA, *IRF-1* mRNA lacking exon 2 (Δ2mRNA), and *IRF-1* mRNA lacking exons 2 and 3 (Δ23mRNA), respectively. The Δ2 and Δ23 mRNAs are also expressed in HL-60 and HEL cells. In addition, a mouse NIH 3T3-derived cell line R27-3 (Harada et al., 1993), which contains a transfected 19 kb human *IRF-1* gene, also showed the same pattern of exon skipping, indicating that this exon skipping occurs independent of the host cell type and chromosomal location. In contrast, when similar RT-PCR analysis was performed for the mouse *IRF-1* mRNA using mouse specific primers, only single band representing the intact mRNA was amplified in R27-3 and mouse hematopoietic cell line BAF/B03 (Figure 1D). Thus, the observed exon skipping may be an intrinsic property of the human *IRF-1* gene. Importantly, the loss of exon 2 in the Δ2 and Δ23 mRNAs should result in the lack of the genuine initiator AUG sequence (Figure 1A, Harada et al., 1994) (see below).
¹ Another 9 samples from healthy donors were examined, and they all essentially showed expression pattern similar to that observed in this case.

### Acceleration of IRF-1 mRNA exon skipping in MDS/leukemia

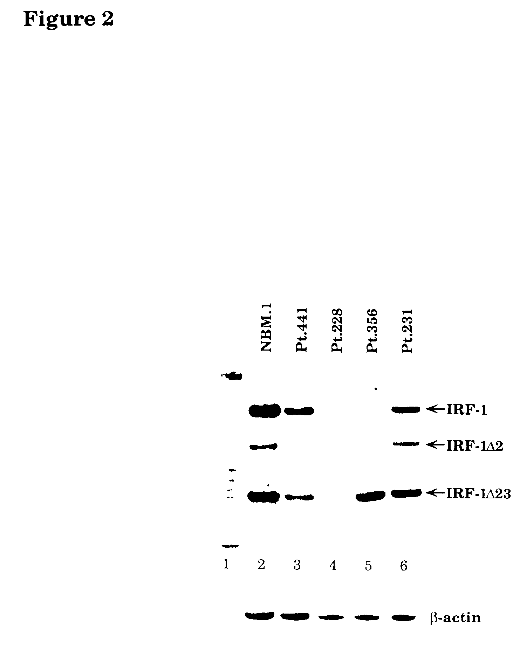

It has been shown that IRF-1 functions as a tumor suppressor (Harada et al., 1993; Tanaka et al., 1994), and deletion and/or rearrangement of either one or both of the *IRF-1* alleles may be critical for the development of human MDS and leukemias (Willman et al., 1993). On the other hand, only a certain proportion of these hematopoietic disorders are accompanied by the 5q anomaly (Kerim et al., 1990; Pederson-Bjergaard et al., 1990; Willman et al., 1993). We examined the status of exon skipping in cells from patients with MDS or leukemias secondary to MDS. Total RNA was isolated from either bone marrow (BM) cells or PBM cells and subjected to the RT-PCR assay. The results of some typical examples are shown in Figure 2. A striking difference is obvious between the BM cells from the healthy donor and those from MDS/leukemia patients. In fact, the amplicon representing the intact *IRF-1* mRNA is barely detectable in two of the samples presented here (Pts. 228 and 356), whereas the amplicon for Δ23 mRNA is still detectable. In contrast, the amount of β-*actin* mRNA specific amplicon remained relatively constant (see Table 1).

We performed a similar analysis for a total of 25 RNA samples from patients with MDS or leukemias secondary to MDS, and the results are summarized in Table 1. The amounts of the IRF specific amplicons were first quantitatively determined by image analysis (Fujix Bas 2000) and then normalized to the amount of β-*actin* amplicon from the same sample; the expression of the β-*actin* mRNA is relatively invariant among the sample RNAs (Makino et al., 1990; Foley et al., 1993). These numbers were further normalized with respect to the levels of *IRF-1* and *IRF-2* amplicons (arbitrarily set to 1.00) found in NBM.1 cells from a healthy donor. Thus, the number appearing in Table 1 represent the expression of the intact, Δ2, Δ23 *IRF-1* mRNAs and *IRF-2* mRNA relative to those of a healthy donor, although they do not provide information regarding absolute mRNA copy number.

Notably, the amplicon representing intact *IRF-1* mRNA was not detected in RNA samples from Pts. 581 and 117 cells, neither of which showed cytogenetic aberrations in the 5q region (Table 1)². In addition, another 5 samples (Pts. 190, 356, 228, 255, 578) gave very low levels of the intact amplicon, whereas the Δ2 and Δ23 amplicons, particularly the Δ 23 amplicon, were still detectable. Six out of these patient samples were also analyzed for p53 mutations by RT-PCR/SSCP analysis, and all except one (Pt. 356) showed no evidence for such mutation (Table 1; Sugimoto et al., 1993). Although not as profound as observed in the above samples, several other samples (e.g. Pts. 570, 716, 194, 199, 707) also show noticeably lower levels of the intact *IRF-1* mRNA amplicon. We also analyzed RNA from patients with different types of hematopoietic malignancies, and found little or no intact *IRF-1* amplicon in some of these samples, including 2 out of 4 AML patients who expressed only the Δ23 mRNA. It may be worth noting that some RNA samples showed elevated levels of the *IRF-2* specific amplicon (e.g. Pts. 581, 199, 534, 715).
² Thus far, RT-PCR/SSCP analysis of four patients with MDS or leukemia which showed an acceleration of exon skipping (Pts. 255, 356, 707 and 716) did not reveal any DNA deletions or mutations within *IRF-1* exon sequences, nor did DNA sequencing reveal any alteration of intron sequences known to affect splicing. In addition, Southern blotting analysis of the DNAs of these patients showed no sign of gross rearrangements of the *IRF-1* gene.

### Analysis of the Δ2 and Δ23 mRNA products

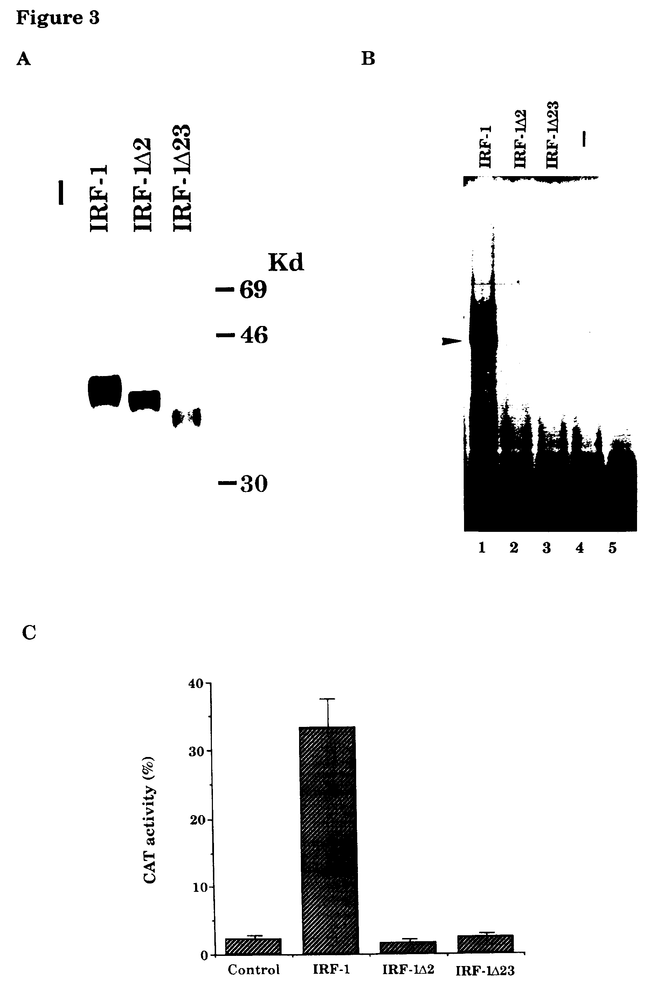

Although both the Δ2 and Δ23 mRNAs lack the genuine initiation codon for *IRF-1*, these mRNAs may still direct synthesis of new proteins. cDNAs were constructed which correspond to the Δ2 or Δ23 mRNAs, and used to direct an *in vitro* transcription-translation reaction. When the ³⁵S methionine-labeled products were analyzed by SDS-PAGE, distinct bands were detected for the wild type, the Δ2 and Δ23 cDNAs (Figure 3A). The size of the Δ2 and Δ23 cDNA products, termed IRF-1Δ2 and IRF-1Δ23, may correspond to truncated IRF-1 proteins whose synthesis had initiated at the AUG codons 32 (in exon 3) and 85 (in exon 4), respectively. As shown in Figure 3B, gel shift assay revealed that both IRF-1Δ2 and IRF-1Δ23 fail to bind to an oligomer containing two high-affinity IRF-Es (C1 oligomer; Tanaka et al., 1993). Co-transfection of each cDNA into a IRF-negative cell line (P19; Harada et al., 1990) along with a reporter plasmid containing IRF-Es in its promoter showed that the protein encoded by the intact *IRF-1* cDNA, but not those encoded by Δ2 or Δ23 cDNAs, could activate the reporter (Figure 3C). Co-transfection of the Δ2 or Δ23 cDNAs with the intact *IRF-1* cDNA did not affect IRF-1-mediated gene activation, suggesting that neither IRF-1Δ2 nor IRF-1Δ23 acts in a dominant-negative manner on IRF-1-mediated transcriptional activation.

### The intact human IRF-1 but not IRF-1Δ23 shows tumor suppressor activity

In most of the samples in which expression of intact *IRF-1* mRNA is low or undetectable, the Δ23 mRNA is nevertheless expressed at relatively high levels (Table 1). We therefore addressed the issue of whether the IRF-1Δ23 manifests tumor suppressor activity. IRF-1^{-/-} EFs expressing an activated form of c-Ha-*ras* oncogene (Tanaka et al., 1994) were transfected with expression vectors containing the cDNA encoding either intact IRF-1 or IRF-1Δ23, together with the hygromycin (*hgr*) resistance gene (pMiwhph; Kato et al., 1990). The transfectants were plated on methylcellulose gel, and the number of *hgr*-resistant colonies was subsequently counted. As shown in Table 2, transfection of the cDNA for IRF-1 resulted in a profound inhibition of colony formation, whereas no such effect was seen with the cDNA for IRF-1Δ23. These results indicate that IRF-1Δ23 lacks of tumor suppressor activity, presumably as the result of the loss of its DNA binding activity.

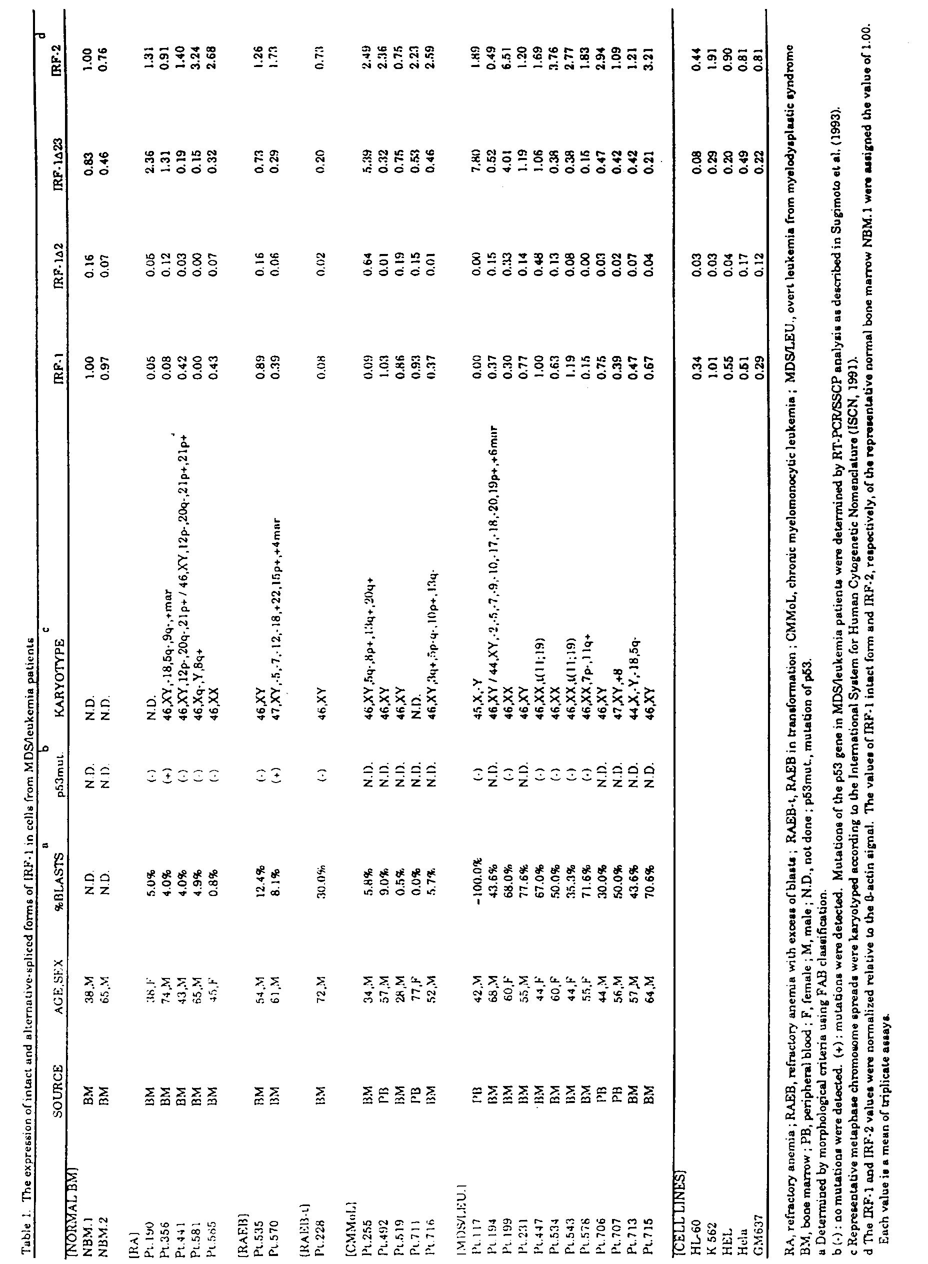

**Table 2**

| Suppression of colony-forming ability by the intact or an alternative splicing form of IRF-1. | | | |
|---|---|---|---|
| Transfected Construct | Number of colonies | | |
| | Experiment 1 | Experiment 2 | Experiment 3 |
| pAct-C | 995 | 934 | 699 |
| pAct-H1 | 381 | 246 | 353 |
| pAct-H1Δ23 | 844 | 803 | 813 |
| Transfected constructs are as follows: pAct-C, control actin promoter vector; pAct-H1, actin promoter expressing intact *IRF-1* cDNA; pAct-H1Δ23, actin promoter expressing *IRF-1* cDNA lacking exons 2 and 3. | | | |

### Examples

### RNA Isolation, S1 Mapping Analysis, and RNA Blotting Analysis

Total cellular RNA was isolated by the guanidinium-thiocyanate method S1 mapping analysis was performed as previously described (Fujita et al., 1987). To prepare probe DNA, pBluescript II SK(+) phage DNA containing the sense strand of the human *IRF-1* cDNA FspI-KpnI fragment was used as template to synthesize ³²P-labeled antisense DNA. The product was then digested by BamHI and probe DNA containing from nucleotide residue 217 to 464 (Maruyama et al., 1989) was isolated. The procedure for RNA blotting analysis is described in Harada et al. (1990). To prepare probe DNA, a 2.0 kb BamHI-PvuII fragment of 1Ha-204 (Miyamoto et al., 1988) for β-*actin* was labeled by the random primer method (Amersham).

### Synthetic Primers

The primers were designed to amplify the cDNA specifically. The primers of the human *IRF-1* gene (Maruyama et al., 1989) were: the antisense primer for reverse transcription (RT), nucleotide (nt) 490 to 471; the sense primer for PCR, nt 92 to 111; the anti-sense primer for PCR, nt 470 to 451. The primers of *IRF-2* gene (Itoh et al., 1989) were: the antisense primer for RT, nt 391 to 372; the sense primer for PCR, nt 19 to 38; the anti-sense primer for PCR, nt 367 to 348. The primers of β-*actin* gene (Ponte et al., 1984) were: the antisense primer for RT, nt 470 to 451; the sense primer for PCR nt 311 to 330; the antisense primer for PCR, nt 448 to 429. The primers of the mouse *IRF-1* gene (Miyamoto et al., 1988) were: the antisense primer for RT, nt 498 to 479; the sense primer for PCR, nt 105 to 124; the antisense primer for PCR, nt 478 to 459.

### RT-PCR Analysis

The RT-PCR analysis was performed basically as previously described (Makino et al. 1990). High specific labeling and fewer cycles of amplification are thought to provide higher sensitivity and clearer results, since the amplicons can be quantitatively detected at a stage where the PCR reaction follows first-order exponential kinetics (Wang et al., 1989; Makino et al., 1990; Foley et al., 1993). In this assay, *IRF-1*, *IRF-2* and β-*actin* cDNAs from the same RNA samples were subjected to PCR amplification, and the β-*actin* amplicon was used as a reference for mRNA quantitation (Makino et al., 1990).

Total RNA was isolated by single-step method described in Chomczynski and Sacchi (1987). Five hundred nanograms of total RNA were added in a reaction mixture containing 1 pmol RT primer, 3.8 ml of 5xRT buffer (250 mM Tris-HCl [pH 8.3], 375 mM KCl, 15 mM MgCl₂) in a volume of 12.5 ml. The mixture was heated at 95°C for 2 min., chilled on ice, and incubated at 37°C for 30 min. After the annealing reaction, the mixture was supplemented with 0.2 ml of 5xRT buffer, 2 ml of 0.1 M DTT, 4.0 ml of 2.5 mM each of dNTPs, 20U of RNbase inhibitor (Takara), and 100U of Moloney murine leukemia virus (M-MLV) reverse transcriptase (GIBCO BRL) to a volume of 20 ml, then incubated at 37°C for 60 min. The RT reaction products were then incubated at 90°C for 5 min, chilled on ice, and added with 40 ml of distilled water. The PCR reaction was performed in a reaction mixture containing 10 mM Tris-HCl (pH 8.3), 50 mM KCl, 1.5 mM MgCl₂, 0.01% (w/v) gelatin, 200 ml of dNTPs, 1 mM each 5' and 3' ³²P-end-labeled PCR primers, 0.25 U Taq DNA polymerase (Perkin-Elmer Cetus), and 3 ml of the RT reaction product in a volume of 10 ml. The amplification was performed with the Perkin-Elmer Cetus DNA thermal cycler as follows: 95°C for 30 sec, 55°C for 30 sec, 72°C for 1 min. The cycles of reaction were determined in the exponential phase of amplification; 24 cycles for *IRF-1* and *IRF-2*, 18 cycles for β-actin. One microliter of each PCR reaction mixture was electrophorased in 5% polyacrylamide gel in 0.5xTBE buffer. The gels were dried and the radioactivity of the appropriate bands were quantified by the Fujix Bas 2000 imaging analyzer. The amount of *IRF-1* and *IRF-2* specific amplicon was normalized to the amount of β-*actin* amplicon from the same sample. These numbers were further normalized with respect to the levels of *IRF-1* and *IRF-2* amplicons (arbitrarily set to 1.00) found in NBM. 1 cells from a healthy donor. In the case of *IRF-1* amplicon, multiple different mRNA species can be quantitated with the same primers as described in Wang et al. (1989).

### Construction of Plasmids

The above RT-PCR products each containing intact or alternative forms of *IRF-1* cDNA were subcloned into pCR™II (Invitrogen). For construction of the plasmids using *in vitro* transcription (pBH1, pBH1Δ2, and pBH1Δ23), the following DNA fragments were ligated; (i) EcoRI-BamHI backbone fragment from pBluescript II SK(+) (ii) EcoRI-EcoRV fragment from the pCR™II derivatives containing *IRF-1* cDNA (iii) EcoRV-BamHI fragment from pHIRF31 (Maruyama et al., 1989). The IRF-1 expression vectors under control of the actin promoter (pAct-H1, pAct-H1Δ2, and pAct-H1Δ23) were constructed by replacing HindIII-BamHI backbone fragment of the pBluescript derivatives with HindIII-BamHI backbone fragment of pAct-C (Harada et al., 1990).

### In Vitro Transcription and Translation

Capped synthetic mRNA was transcribed in the presence of cap analog and ribonucleotide triphosphate by T7 RNA polymerase with the T7 promoter of each derivertive of pBluescript II containing the intact or mutant *IRF-1* cDNAs (pBH1, pBH1Δ2, or pBH1Δ 23). The reaction was performed as the manufacturer's protocol (Stratagene). The RNA was extracted with phenol-chloroform and ethanol precipitated. The RNA was redissolved in water, and 0.8 mg of the RNA was added in *in vitro* translation, using rabbit reticulocyte lysate (Amersham) at 30°C for 60 min.

### Gel Shift Assay

The assay was performed essentially as previously described (Harada et al., 1990). ³²P-labeled C1 oligomer (containing two IRF binding motifs; Tanaka et al., 1993) was used as probe DNA.

### DNA Transfection and CAT Assay

P19 embryonal carcinoma cells (2.5x10⁵ cells/6 cm dish) were transfected with 5 mg of p-55C1B reporter gene, which contains four IRF binding motifs (Fujita et al., 1987; Tanaka et al., 1993), and 5 mg of the IRF-1 expression vector. CAT assay was carried out as described (Harada et al., 1990).

### Colony-Forming Assay in Methylcellulose Gel

The IRF-1^{-/-} embryonic fibroblast cells expressing an activated form of c-Ha-ras, rasEF11 (Tanaka et al., 1994) (5x10⁵ cells/10 cm dish), were co-transfected with 15 mg of the IRF-1 expression vector and 0.3 mg of the pMiwhph (Kato et al., 1988) by the calcium phosphate method (Harada et al., 1990). Seventy-two hours alter the transfection, cells were suspended with 1.3% methylcellulose gel dissolved in culture medium containing 200 mg/ml hygromycin and overlayed on an agarose bed composed of 0.53% agarose and culture medium. Colonies were scored 3 weeks alter plating.

### Figure legends

### Figure 1

(A) The alternative splicing of human IRF-1 pre-mRNA. In the top panel exons are numbered as indicated, and lines connecting the exons represent splicing. The initiator ATG sequence within exon 2 is indicated. In the lower panel the intact and the alternative splicing forms of IRF-1 DNA are shown. The expected sizes of the probes protected from S1 digestion and the products of RT-PCR amplification are indicated.
(B) Detection of the alternative splicing forms of IRF-1 pre-mRNA by S1 mapping analysis. Total RNA was isolated from peripheral blood mononuclear (PBM) cells of a healthy volunteer (NPB.1), HL-60 cells, and HEL cells. Upper panel; 15 mg of the RNAs were subjected to S1 mapping analysis (see Experimental Procedures). Lane 1, ³²P-labeled HaeIII-digested pBR322 DNA fragments; lane 2, yeast tRNA as a negative control; lane 3, PBM cells from a healthy donor (NPB.1); lane 4, HL-60; lane 5, HEL. The arrows indicate the positions of protected probes as described in (A). Lower panel; 2.5 mg of the RNAs were subjected to Northern blot analysis and the filter was hybridized with a β-actin probe.
(C) RT-PCR analysis of IRF-1, IRF-2, and β-actin mRNA in PBM cells from a healthy donor, HL-60, and HEL. Five hundred nanograms of total RNA were reverse-transcribed into cDNA using primers specific for IRF-1, IRF-2 and β-actin, respectively. The cDNA product was subjected to PCR with labeled primers (see Experimental Procedures). Lane 1, yeast tRNA as a negative control; lane 2, PBM cells from a healthy donor (NPB. 1); lane 3, HL-60; lane 4, HEL; lane 5, ³²P-labeled HaeIII-digested pBR322 DNA fragments. In the upper panel, the arrows indicate the positions of PCR products as described in (A). In the middle and lower panel the IRF-2 and β-actin specific amplicons, respectively, are shown.
(D) RT-PCR analysis of the human versus murine IRF-1 mRNAs. Total RNAs from an NIH 3T3-derived clone transfected with the human IRF-1 genomic DNA (R27-3; Harada et al., 1993) and from mouse BAF/B03 were subjected to RT-PCR analysis using human or mouse IRF-1 specific primers (see Experimental procedures). The arrows indicate the positions of PCR products as described in (A). Lane 1, ³²P-labeled HaeIII-digested pBR322 DNA fragments; lane 2, human IRF-1 amplicon in NIH 3T3-derived clone (R27-3); lane 3, mouse IRF-1 amplicon in R27-3; lane 4, mouse IRF-1 amplicon in BAF/B03.

### Figure 2: RT-PCR analysis of mRNA from representative MDS/leukemia patients.

RT-PCR was performed with total RNAs from a healthy donor and from MDS/leukemia patients (see Experimental Procedures). In the upper panel the arrows indicate the positions of the PCR products as described in Figure 1A. Lane 1, ³²P-labeled HaeIII-digested pBR322 DNA fragments; lane 2, bone marrow cells from a healthy donor (NBM.1); lane 3, Pt.441; lane 4, Pt.228; lane 5, Pt.356; lane 6, Pt.231. In the lower panel, β-actin specific amplicons are shown.

### Figure 3

(A) *In vitro* translated products of the intact and alternative forms of IRF-1. One microliter of each translation reaction (10 ml) was analyzed on a 12.5% SDS-polyacrylamide gel.
(B) The DNA binding activity of the IRF-1 mutants. The *in vitro* translated proteins shown in (A) were subjected to gel shift assay. Lane 4, *in vitro* translated product without RNA; lane 5, no extract. The arrowhead indicates the position of the IRF-1-DNA complex.
(C) Transcriptional activation by the IRF-1 mutants. P19 cells were transfected with 5 mg of the CAT reporter gene p-55C1B and 5 mg of the effector genes. The transfected effector genes were as follows: control, 5 mg of pAct-C; IRF-1, 2.5 mg of pAct-H1 and 2.5 mg of pAct-C; IRF-1Δ2, 2.5 mg of pAct-H1Δ2 and 2.5 mg of pAct-C; IRF-1Δ23, 2.5 mg of pAct-H1Δ23 and 2.5 mg of pAct-C. The transfection was duplicated and the assay was repeated three times; results were essentially reproducible.

### References

Abdollahi, A., Lord, K. A., Hoffman-Liebermann, B., and Liebermann, D. A. (1991). Interferon regulatory factor 1 is a myeloid differentiation primary response gene induced by interleukin 6 and leukemia inhibitory factor: Role in growth inhibition. Cell Growth Differ. *2*, 401-407.

Au, W.-C., Raj, N. B. K., Pine, R., and Pitha, P. M. (1992). Distinct activation of murine interferon-a promoter region by IRF-1/ISGF-2 and virus infection. Nucleic acids Res. *20,* 2877-2884.

Boultwood, J., Fidler, C., Lewis, S., MacCarthy, A., Sheridan, H., Kelly, S., Oscier, D., Buckle, V. J., and Wainscoat, J. S. (1993). Allelic loss of IRF1 in myelodysplasia and acute myeloid leukemia: Retention of IRF1 on the 5q- chromosome in some patients with the 5q-syndrome. Blood *82*, 2611-2616.

Carter, G., Rigde, S., and Padua, R. A. (1992). Genetic lesions in preleukemia. Crit. Rev. Oncog. *3*, 339-364.

Cha, Y., Sims, S. H., Romine, M. F., Kaufmann, M., and Deisseroth, A. B. (1993). Human interferon regulatory factor 1: Intron-exon organization. DNA Cell Biol. *11*, 605-611.

Chomczynski, P., and Sacchi, N. (1987). Single-step method of RNA isolation by acid guanidinium thiocyanate-phenol-chloroform extraction. Anal. Biochem. *162*, 156-159.

Foley, K. P., Leonard, M. W., and Engel, J. D. (1993). Quantitation of RNA using the polymerase chain reaction. Trens Genet. *9*, 380-385.

Friedman, R. L., and Stark, G. R. (1985). a-Interferon-induced transcription of HLA and metallothionein genes containing homologous upstream sequences. Nature *314*, 637-639.

Fujita, T., Shibuya, H., Hotta, H., Yamanishi, K., and Taniguchi, T. (1987). Interferon-b gene regulation: Tandemly repeated sequences of a synthetic 6 bp oligomer function as a virus-inducible enhancer. Cell *49*, 357-367.

Fujita, T., Kimura, Y., Miyamoto, M., Barsoumian, E. L., and Taniguchi, T. (1989). Induction of endogenous IFN-α and IFN-β genes by a regulatory transcription factor, IRF-1. Nature *337*, 270-272.

Gallagher, R., Collins, S., Trujillo, J., McCredie, K., Ahearn, M., Tsai, S., Metzgar, R., Aulakh, G., Ting, R., Ruscetti, F., and Gallo, R. (1979). Characterization of the continuous, differentiating myeloid cell line (HL-60) from a patient with acute promyelocytic leukemia. Blood *54*, 713-733.

Haber, D. A., and Housman, D. E. (1992). The genetics of Wilms' tumor. Adv. Cancer Res. *59*, 41-68.

Harada, H., Fujita, T., Miyamoto, M., Kimura, Y., Maruyama, M., Furia, A., Miyata, T., and Taniguchi, T. (1989). Structurally similar but functionally distinct factors, IRF-1 and IRF-2, bind to the same regulatory elements of IFN and IFN-inducible genes. Cell *58*, 729-739.

Harada, H., Willison, K., Sakakibara, J., Miyamoto, M., Fujita, T., and Taniguchi, T. (1990). Absence of the type I IFN system in EC cells: Transcriptional activator (IRF-1) and repressor (IRF-2) genes are developmentally regulated. Cell *63*, 303-312.

Harada, H., Kitagawa, M., Tanaka, N., Yamamoto, H., Harada, K., Ishihara, M., and Taniguchi, T. (1993). Anti-oncogenic and oncogenic potentials of interferon regulatory factors-1 and -2. Science *259*, 971-974.

Harada, H., Takahashi, E., Itoh, S., Harada, K., Hori, T., and Taniguchi, T. (1994). Structure and regulation of the human interferon regulatory factor 1 (IRF-1) and IRF-2 genes: Implications for a gene network in the interferon system. Mol. Cell. Biol. *14*, 1500-1509.

Itoh, S., Harada, H., Fujita, T., Mimura, T., and Taniguchi, T. (1989). Sequence of a cDNA coding for human IRF-2. Nucleic Acids Res. *17*, 8372.

Itoh, S., Harada, H., Nakamura, Y., White, R., and Taniguchi, T. (1991). Assignment of the human interferon regulatory factor-1 (IRF1) gene to chromosome 5q23-31. Genomics *10*, 1097-1099.

Kaelin, Jr., W. G., Pallas, D. C., DeCapiro, J. A., Kaye, F. J., and Livingston, D. M. (1991). Cellular proteins that can interact specifically with the retinoblastoma susceptibility gene product. *In* Origin of human cancer: A comprehensive review, Brugge, J., Curran, T., Harlow, E., McCormik, F. eds. (Cold Spring Harbor Laboratory Press), pp.423-431.

Kamijo, R., Harada, H., Matsuyama, T., Bosland, M., Gerecitano, J., Shapiro, D., Le, J., Koh, S. I., Kimura, T., Green, S. J., Mak, T. W., Taniguchi, T., and Vilcek, J. (1994). Requirement for transcription factor IRF-1 in NO synthase induction in macrophages. Science, in press.

Kato, K., Kanamori, A., and Kondoh, H. (1990). Rapid and transient decrease of N-*myc* expression in retinoic acid-induced differentiation of OTF9 teratocarcinoma stem cells. Mol. Cell. Biol. *10*, 486-491.

Kerim, S., Mecucci, C., Cuneo, A., Vandenberghe, E., Louwagie, A., Stul, M., Michaux, J.-L., and Van den Berghe, H. (1990). 5q- anomaly in lymphoid disorders. Leukemia *4*, 12-15.

Kirchhoff, S., Schaper, F., and Hauser, H. (1993). Interferon regulatory factor 1 (IRF-1) mediates cell growth inhibition by transactivation of downstream target genes. Nucleic Acids Res. *21,* 2881-2889.

Le Beau, M. M., Albain, K. S., Larson, R. A., Vardiman, J. W., Davis, E. M., Blough, R. R., Golomb, H. M., and Rowley, J. D. (1986). Clinical and cytogenetic correlations in 63 patients with therapy-related myelodysplastic syndromes and acute nonlymphocytic leukemia: Further evidence for characteristic abnormalities of chromosomes No. 5 and 7. J. Clin. Oncol. *4*, 325-345.

Le Beau, M. M., Chandrasekharappa, S. C., Lemons, R. S., Schwartz, J. L., Larson, R. A., Arai, N., and Westbrook, C. A. (1989). Molecular and cytogenetic analysis of chromosome 5 abnormalities in myeloid disorders: Chromosomal localization and physical mapping of IL-4 and IL-5. Cancer Cells *7*, 53-58.

Le Beau, M. M., Espinosa III, R., Neuman, W. L., Stock, W., Roulston, D., Larson, R. A., Keinanen, M., and Westbrook, C. A. (1993). Cytogenetic and molecular delineation of the smallest commonly deleted region of chromosome 5 in malignant myeloid diseases. Proc. Natl. Acad. Sci. USA *90*, 5484-5488.

Levine, A. J. (1993). The tumor suppressor genes. Annu. Rev. Biochem. 62, 623-651.

Levy, D. E., Kessler, D. S., Pine, R., Reich, N., and Darnell, Jr., J. E. (1988). Interferon-induced nuclear factors that bind a shared promoter element correlate with positive and negative transcriptional control. Genes Dev. *2*,383-393.

Lewin, B. (1991). Oncogenic conversion by regulatory changes in transcription factors. Cell *64*,303-312.

Makino, R., Sekiya, T., and Hayashi, K. (1990). Evaluation of quantitative detection of mRNA by the reverse transcription-polymerase chain reaction. Technique *2*, 295-301.

Marshall, C. J. (1991). Tumor suppressor genes. Cell *64*, 313-326.

Maruyama, M., Fujita, T., and Taniguchi, T. (1989). Sequence of a cDNA coding for human IRF-1. Nucleic Acids Res. *17*, 3292.

Matsuyama, T., Kimura, T., Kitagawa, M., Pfeffer, K., Kawakami, T., Watanabe, N., Kündig, T. M., Amakawa, R., Kishihara, K., Wakeham, A., Potter, J., Furlonger, C. L., Narendran, A., Suzuki, H., Ohashi, P. S., Paige, C. J., Taniguchi, T., and Mak, T. W. (1993). Targeted disruption of IRF-1 or IRF-2 results in abnormal type I IFN gene induction and aberrant lymphocyte development. Cell *75*, 83-97.

Miyamoto, M., Fujita, T., Kimura, Y., Maruyama, M., Harada, H., Sudo, Y., Miyata, T., and Taniguchi, T. (1988). Regulated expression of a gene encoding a nuclear factor, IRF-1, that specifically binds to IFN-β gene regulatory elements. Cell *54*, 903-913.

Nimer, S. D., and Golde, D. W. (1987). The 5q- Abnormality. Blood *70*, 1705-1712.

Pederson, B., and Jensen, I. M. (1991). Clinical and prognostic implications of chromosome 5q deletions: 96 high resolution studied patients. Leukemia *5*, 566-573.

Pedersen-Bjergaard, J., Philip, P., Larsen, S. O., Jensen, G., and Byrsting, K. (1990). Chromosome aberrations and prognostic factors in therapy-related myelodysplasia and acute nonlymphocytic leukemia. Blood *76*, 1083-1091.

Pine, R., Decker, T., Kessler, D. S., Levy, D. E., and Darnell, Jr., J. E. (1990). Purification and cloning of interferon-stimulated gene factor 2 (ISGF2): ISGF2 (IRF-1) can bind to the promoters of both beta interferon- and interferon-stimulated genes but is not a primary transcriptional activator of either. Mol. Cell. Biol. *10*, 2448-2457.

Pine, R. (1992). Constitutive expression of an ISGF2/IRF1 transgene leads to interferon-independent activation of interferon-inducible genes and resistance to virus infection. J. Virol. *66*, 4470-4478.

Ponte, P., Ng, S.-Y., Engel, J., Gunning, P., and Kedes, L. (1984). Evolutionary conservation in the untranslated regions of actin mRNAs: DNA sequence of a human beta-actin cDNA. Nucleic Acids Res. *12*, 1687-1696.

Reis, L. F. L., Harada, H., Wolchok, J. D., Taniguchi, T., and Vilcek, J. (1992). Critical role of a common transcription factor, IRF-1, in the regulation of IFN-β and IFN-inducible genes. EMBO J. *11*, 185-193.

Ruffner, H., Reis, L. F. L., Näf, D., and Weissmann, C. (1993). Induction of type I interferon genes and interferon-inducible genes in embryonic stem cells devoid of interferon regulatory factor 1. Proc. Natl. Acad. Sci. USA *90*, 11503-11507.

Shirayoshi, Y., Miyazaki, J., Burke, P. A., Hamada, K., Appella, E., and Ozato, K. (1987). Binding of multiple nuclear factors to the 5' upstream regulatory element of the murine major histocompatibility class I gene. Mol. Cell. Biol. *7*, 4542-4548.

Stark, G. R., and Kerr, I. M. (1992). Interferon-dependent signaling pathways: DNA elements, transcription factors, mutations, and effects of viral proteins. J. Interferon Res. *12*, 147-151.

Sugimoto, K., Hirano, N., Toyoshima, H., Chiba, S., Mano, H., Takaku, F., Yazaki, Y., and Hirai, H. (1993). Mutations of the p53 gene in myelodysplastic syndrome (MDS) and MDS-derived leukemia. Blood *81*, 3022-3026.

Tanaka, N., Ishihara, M., Kitagawa, M., Harada, H., Kimura, T., Matsuyama, T., Aizawa, S., Mak, T. W., and Taniguchi, T. (1994). Cellular commitment to oncogene-induced transformation or apoptosis is dependent on the transcription factor IRF-1. Accompanied paper.

Tanaka, N., Kawakami, T., and Taniguchi, T. (1993). Recognition DNA sequences of interferon regulatory factor 1 (IRF-1) and IRF-2, regulators of cell growth and the interferon system. Mol. Cell. Biol. *13*, 4531-4538.

Tanaka, N., and Taniguchi, T. (1992). Cytokine gene regulation: Regulatory *cis*-elements and DNA binding factors involved in the interferon system. Adv. Immunol. *52*, 263-281.

Tanaka, S., Liu, L., Kimura, J., Shiojiri, S., Takahashi, Y., Kitaguchi, N., Nakamura, S., and Ueda, K. (1992). Age-related changes in the proportion of amyloid precursor protein mRNAs in Alzheimer's disease and other neurological disorders. Mol. Brain Res. *15*, 303-310.

Van den Berghe, H., Vermaelen, K., Mecucci, C., Barbieri, D., and Tricot, G. (1985). The 5q- anomaly. Cancer Genet. Cytogenet. *17*, 189-255.

Vogelstein, B., and Kinzler, K. W. (1992). p53 function and dysfunction. Cell *70*, 523-526.

Wang, A. M., Doyle, M. V., and Mark, D. F. (1989). Quantitation of mRNA by the polymerase chain reaction. Proc. Natl. Acad. Sci. USA *86*, 9717-9721.

Weinberg, R. A. (1991). Tumor suppressor genes. Science *254*, 1138-1146.

Willman, C. L., Sever, C. E., Pallavicini, M. G., Harada, H., Tanaka, N., Slovak, M. L., Yamamoto, H., Harada, K., Meeker, T. C., List, A. F., and Taniguchi, T. (1993). Deletion of IRF-1, mapping to chromosome 5q31.1, in human leukemia and preleukemic myelodysplasia. Science *259*, 968-971.

Yamada, G., Ogawa, M., Akagi, K., Miyamoto, H., Nakano, N., Itoh, S., Miyazaki, J., Nishikawa, S., Yamamura, K., and Taniguchi, T. (1990). Specific depletion of the B-cell population induced by aberrant expression of human interferon regulatory factor 1 gene in transgenic mice. Proc. Natl. Acad. Sci. USA *88*, 532-536.

Yu-Lee, L.-Y., Hrachovy, J. A., Stevens, A. M., and Schwarz, L. A. (1990). Interferon-regulatory factor 1 is an immediate-early gene under transcriptional regulation by prolactin in Nb2 T cells. Mol. Cell. Biol. *10*, 3087-3094.

## Claims

1. Method of diagnosing cancer or precancerous state characterized in that altered splicing of *IRF-1*-specific RNA is analysed in a sample obtained *in vitro* from blood or other biopsy material wherein it is determined whether the sample contains *IRF-1*-specific RNA molecules which are shortened in comparison to a RNA molecule which codes for the complete amino acid sequence of the *IRF-1* gene.

2. Method of claim 1, characterized in that it is determined whether said *IRF-1*-specific RNA molecules lack exon 2 (*IRF-1Δ2*-RNA) or exons 2 and 3 (*IRF-1Δ23-RNA*) of the *IRF-1* gene.

3. Method of claim 1 or 2, characterized in that the relative amounts of full-length *IRF-1*-RNA, *IRF-1Δ2*-RNA and *IRF-1Δ23-RNA* are determined.

4. Method according to any one of claims 1 to 3, characterized in that said analysis is carried out by reverse transcription/polymerase chain reaction and subsequent analysis of the amplified cDNA.

5. Method according to claim 4, characterized in that at least one of the oligonucleotides
5'TTCCCTCTTCCACTCGGAGT3'(SEQ ID NO: 1),
5'GATATCTGGCAGGGAGTTCA3' (SEQ ID NO: 2), or
5' CTCTGGTCTTTCACCTCCTC3'(SEQ ID NO: 3)
is used as a primer.

6. Method according to any one of claims 1 to 5, characterized in that said cancer or precancerous state is a disease of the hematopoietic system.

7. Method according to claim 6, characterized in that said disease of the hematopoietic system is myelodysplasia or leukemia.

8. Method according to claim 6 or 7, characterized in that said sample is obtained from peripheral blood or bone marrow.

9. Use of at least one *IRF-1*-specific oligonucleotide in a method of diagnosing cancer or precancerous state wherein *IRF-1*-specific RNA is analysed in a sample obtained from blood or other biopsy material and it is determined whether the sample contains *IRF-1*-specific RNA molecules which are shortened in comparison to a RNA molecule which codes for the complete amino acid sequence of the *IRF-1* gene.

10. Use of claim 9, characterized in that said analysis is carried out by reverse transcription/polymerase chain reaction and subsequent analysis of the amplified cDNA.

11. Use according to claims 9 or 10, characterized in that said oligonucleotide has the sequence
5'TTCCCTCTTCCACTCGGAGT3' (SEQ ID NO: 1),
5' GATATCTGGCAGGGAGTTCA3' (SEQ ID NO: 2), or
5'CTCTGGTCTTTCACCTCCTC3'(SEQ ID NO: 3).

12. Oligonucleotide having the sequence 5' TTCCCTCTTCCACTCGGAGT3' (SEQ ID NO: 1),
5' GATATCTGGCAGGGAGTTCA3' (SEQ ID NO: 2), or
5' CTCTGGTCTTTCACCTCCTC3' (SEQ ID NO: 3).

13. Method of diagnosing cancer or precancerous state, characterized in that the content of biologically active IRF-1 polypeptide is determined in a sample which is obtained *in vitro* from blood or other biopsy material wherein it is determined whether the sample contains *IRF-1*-specific polypeptide molecules which are shortened in comparison to a polypeptide molecule comprising the complete amino acid sequence of the *IRF-1* gene.

## Patentansprüche

1. Verfahren zur Diagnose von Krebs oder Präkanzerose, dadurch gekennzeichnet, dass eine in vitro aus Blut oder anderem Biopsiematerial erhaltene Probe nach verändertem Spleissen von *IRF-1*-spezifischer RNA analysiert wird, wobei bestimmt wird, ob die Probe *IRF-1*-spezifische RNA-Moleküle enthält, die im Vergleich zu einem für die vollständige Aminosäure-Sequenz des *IRF-1*-Gens codierenden RNA-Molekül gekürzt sind.

2. Verfahren nach Anspruch 1, dadurch gekennzeichnet, dass bestimmt wird, ob den genannten *IRF-1*-spezifischen RNA-Molekülen das Exon 2 (*IRF-1Δ2*-RNA) oder die Exons 2 und 3 (*IRF-1Δ23*-RNA) des *IRF-1*-Gens fehlen.

3. Verfahren nach Anspruch 1 oder 2, dadurch gekennzeichnet, dass die relativen Mengen von ungekürzter *IRF-1*-RNA, *IRF-1Δ2*-RNA und *IRF-1Δ23*-RNA bestimmt werden.

4. Verfahren nach einem der Ansprüche 1 bis 3, dadurch gekennzeichnet, dass die genannte Analyse mittels Reverse-Transkription/Polymerase-Kettenreaktion und nachfolgender Analyse der amplifizierten cDNA durchgeführt wird.

5. Verfahren nach Anspruch 4, dadurch gekennzeichnet, dass mindestens eines der Oligonucleotide
5'TTCCCTCTTCCACTCGGAGT3' (SEQ ID NR: 1),
5'GATATCTGGCAGGGAGTTCA3' (SEQ ID NR: 2), oder
5'CTCTGGTCTTTCACCTCCTC3' (SEQ ID NR: 3)
als Primer verwendet wird.

6. Verfahren nach einem der Ansprüche 1 bis 5, dadurch gekennzeichnet, dass der genannte Krebs oder die genannte Präkanzerose eine Erkrankung des Hämatopoesesystems ist.

7. Verfahren nach Anspruch 6, dadurch gekennzeichnet, dass die genannte Erkrankung des Hämatopoesesystems eine Myelodysplasie oder Leukämie ist.

8. Verfahren nach Anspruch 6 oder 7, dadurch gekennzeichnet, dass die genannte Probe aus peripherem Blut oder Knochenmark erhalten wird.

9. Verwendung mindestens eines *IRF-1*-spezifischen Oligonucleotids bei einem Verfahren zur Diagnose von Krebs oder Präkanzerose, wobei eine aus Blut oder anderem Biopsiematerial erhaltene Probe nach *IRF-1*-spezifischer RNA analysiert wird und bestimmt wird, ob die Probe *IRF-1*-spezifische RNA-Moleküle enthält, die im Vergleich zu einem für die vollständige Aminosäure-Sequenz des *IRF-1*-Gens codierenden RNA-Molekül gekürzt sind.

10. Verwendung nach Anspruch 9, dadurch gekennzeichnet, dass die genannte Analyse mittels Reverse- Transkription/Polymerase-Kettenreaktion und nachfolgender Analyse der amplifizierten cDNA durchgeführt wird.

11. Verwendung nach Anspruch 9 öder 10, dadurch gekennzeichnet, dass das genannte Oligonucleotid die Sequenz
5'TTCCCTCTTCCACTCGGAGT3' (SEQ ID NR: 1),
5'GATATCTGGCAGGGAGTTCA3' (SEQ ID NR: 2), oder
5'CTCTGGTCTTTCACCTCCTC3' (SEQ ID NR: 3)
aufweist.

12. Oligonucleotid, das die Sequenz
5' TTCCCTCTTCCACTCGGAGT3' (SEQ ID NR: 1) ,
5'GATATCTGGCAGGGAGTTCA3' (SEQ ID NR: 2), oder
5'CTCTGGTCTTTCACCTCCTC3' (SEQ ID NR: 3)
aufweist.

13. Verfahren zur Diagnose von Krebs oder Präkanzerose, dadurch gekennzeichnet, dass der Gehalt an biologisch aktivem *IRF-1*-Polypeptid in einer in vitro aus Blut oder anderem Biopsiematerial erhaltenen Probe bestimmt wird, wobei bestimmt wird, ob die Probe *IRF-1*-spezifische Polypeptid-Moleküle enthält, die im Vergleich zu einem die vollständige Aminosäure-Sequenz des *IRF-1*-Gens enthaltenden Polypeptid-Molekül gekürzt sind.

## Revendications

1. Procédé de diagnostic de cancer ou de prénéoplasie, caractérisé en ce que l'on procède à une analyse de l'altération d'épissage de l'ARN spécifique d'*IRF-1* dans un échantillon obtenu in vitro à partir de sang ou d'une autre matière de biopsie, au cours de laquelle on détermine si l'échantillon contient des molécules d'ARN spécifique d'*IRF∼1* qui sont raccourcies en comparaison d'une molécule d'ARN qui code la séquence complète d'acides aminés du gène *IRF-1*.

2. Procédé selon la revendication 1, caractérisé en ce que l'on détermine s'il manque auxdites molécules d'ARN spécifique d'*IRF-1* l'exon 2 (*IRF-1Δ2-ARN*) ou les exons 2 et 3 (*IRF-1Δ23-ARN*) du gène *IRF-1*.

3. Procédé selon les revendications 1 ou 2, caractérisé en ce que l'on détermine les quantités relatives d'*IRF-1-ARN*, d'*IRF-1Δ2-ARN* et d'*IRF-1Δ23-ARN* non raccourcis.

4. Procédé selon l'une des revendications 1 à 3, caractérisé en ce que l'on procède à ladite analyse par transcription inverse/réaction en chaîne de la polymérase suivie d'une analyse de l'ADNc amplifié.

5. Procédé selon la revendication 4, caractérisé en ce que l'on utilise comme amorce l'un au moins des oligonucléotides
5'TTCCCTCTTCCACTCGGAGT3' (ID SEQ N^{o}: 1),
5'GATATCTGGCAGGGACTTCA3' (ID SEQ N^{o}: 2), ou
5'CTCTGGTCTTTCACCTCCTC3' (ID SEQ N^{o}: 3)

6. Procédé selon l'une des revendications 1 à 5, caractérisé en ce que ledit cancer ou ladite prénéoplasie est une maladie du système hématopoïétique.

7. Procédé selon la revendication 6, caractérisé en ce que ladite maladie du système hématopoïétique est la myélodysplasie ou la leucémie.

8. Procédé selon la revendication 6 ou 7, caractérisé en ce que l'on obtient ledit échantillon à partir de sang périphérique ou de moelle osseuse.

9. Utilisation d'au moins un oligonucléotide spécifique d'*IRF-1* dans un procédé de diagnostic de cancer ou de prénéoplasie au cours duquel l'on procède à une analyse de l'ARN spécifique d'*IRF-1* dans un échantillon obtenu *in vitro* à partir de sang ou d'une autre matière de biopsie et l'on détermine si l'échantillon contient des molécules d'ARN spécifique d'*IRF-1* qui sont raccourcies en comparaison d'une molécule d'ARN qui code la séquence complète d'acides aminés du gène *IRF-1*.

10. Utilisation selon la revendication 9, caractérisée en ce que l'on procède à ladite analyse par transcription inverse/ réaction en chaîne de la polymérase suivie d'une analyse de l'ADNc amplifié.

11. Utilisation selon la revendication 9 ou 10, caractérisée en ce que ledit oligonucléotide comporte la séquence
5'TTCCCTCTTCCACTCGGAGT3' (ID SEQ N^{o}: 1),
5'GATATCTGGCAGGGAGTTCA3' (ID SEQ N^{o}: 2), ou
5'CTCTGGTCTTTCACCTCCTC3' (ID SEQ N^{o}: 3)

12. Oligonucléotide comportant la séquence
5'TTCCCTCTTCCACTCGGAGT3' (ID SEQ N^{o}: 1),
5'GATATCTGGCAGGGACTTCA3' (ID SEQ N^{o}: 2), ou
5'CTCTGGTCTTTCACCTCCTC3' (ID SEQ N^{o}: 3)

13. Procédé de diagnostic de cancer ou de prénéoplasie, caractérisé en ce que l'on détermine la teneur en polypeptide *IRF-1* biologiquement actif dans un échantillon obtenu in vitro à partir de sang ou d'une autre matière de biopsie, au cours duquel on détermine si l'échantillon contient des molécules de polypeptide spécifique d'*IRF-1* qui sont raccourcies en comparaison d'une molécule de polypeptide qui comporte la séquence complète d'acides aminés du gène *IRF-1*.
